# EUROPEAN PATENT SPECIFICATION

(11) **EP 3 426 126 B1**
(45) Date of publication and mention of the grant of the patent: **04.05.2022**
(21) Application number: 17708778.0
(22) Date of filing: 03.03.2017
(51) Int. Cl.: A61L 9/012, A61L 9/04, A61L 9/05, A61L 9/12, D06F 58/20, A47L 15/44

(54) **FRAGRANCE RELEASE SYSTEM**
DUFTFREISETZUNGSSYSTEM
SYSTÈME DE LIBÉRATION DE PARFUM

(30) Priority: 10.03.2016 DE 102016203988
(43) Date of publication of application: 16.01.2019
(73) Proprietor: Henkel AG & Co. KGaA, 40589 Düsseldorf (DE)
(72) Inventor: RISTAU, Steffen, 66620 Nonnweiler (DE); WOLTER, Bernd, 41468 Neuss (DE); HENNING, Ingomar, 51588 Nümbrecht (DE); GERHARDS, Katja, 40547 Düsseldorf (DE); WARDEN, Bodo, 41061 Mönchengladbach (DE)
(86) International application number: PCT/EP2017/055052
(87) International publication number: WO 2017/153287

(56) References cited:
- WO-A1-02/09779
- WO-A1-96/38638
- US-A- 4 995 556
- US-A1- 2005 148 479
- US-A1- 2009 088 357

## Description

The invention relates to a fragrance release system comprising a container and a multitude of particles stored in the chamber of the container for deodorizing and/or fragrancing open or closed spaces, especially machine dishwasher textile washing machines or laundry dryers, said particles having a preferably polymeric carrier material and at least one fragrance, and said container having a plurality of orifices through which emission of the fragrances of the particles from the chamber outward is possible.

In closed spaces without sufficient fresh air supply, unpleasant odors frequently occur. Such spaces may, for example, be the interiors of machine dishwashers when the dishes present there are very highly soiled and/or remain in the dishwasher over a prolonged period before the washing operation. In order to remove or to reduce such odors, machine dishwasher deodorants are known for machine dishwashers. These deodorants may be formulated in very different ways. For the consumer, it is desirable to obtain an article for deodorizing machine dishwashers or other closed spaces which has an intense product fragrance when it is produced, which not only ensures product identification, but simultaneously conveys the impression of high efficacy and ensures a very reliable release of constant amounts of fragrance in the course of its lifetime. A series of different deodorants for machine dishwashers is described in the prior art.

A fragrance release system as per the preamble of claim 1 is known from WO 02/09779 A1. This known fragrance release system has a container in which a multitude of small particles laden with fragrances is stored. The container is provided with a multitude of orifices whose size is dimensioned such that the small particles cannot escape through the orifices. On the other hand, the orifices are dimensioned such that emission of the fragrances of the particles from the chamber of the container outward is possible. The container itself preferably has an approximately cuboidal shape, with rounded corners and an openable and closable lid. The chamber of the container may be divided into two sections in which perfumed particles are disposed. The particles themselves consist preferably of a polymeric carrier material which is laden with a fragrance.

In the known fragrance release system, the container is filled with particles preferably between 5 and 95% of the volume of the chamber, because a maximum particle volume but equally free mobility of the particles should be achieved.

However, the known fragrance release system is afflicted with disadvantages, some of which can be attributed to the design of the container, since the container shape described is suited very little, for example, to use in a machine dishwasher or the like, since the container is relatively large.

Another disadvantage is that it is virtually impossible in the case of the selected container shape to virtually fully fill the chamber of the container with particles, which is desirable because mobility of the particles is undesired especially in the case of use within a machine dishwasher, since virtually the entire surface area of all particles is simultaneously available for fragrance release and the functioning time of the system is limited.

Other solutions for the deodorization and/or fragrancing of washing basins or else machine dishwashers are known from DE 100 55 193 A1, DE 100 36 850 A1, WO2004020003, and WO2004020004 to the applicant.

Fragrance-containing articles made of ethylene/vinyl acetate copolymer are disclosed in the publication WO 91/00744 A1. These are injection-molded plastics plaques which can be hung in the machine dishwasher. Since the fragrance is mixed with the plastic before the injection molding, it is exposed to high thermal stress during the processing. Such thermal stress leads to a partial loss of fragrances as a result of evaporation or thermal decomposition. Such plastics plaques likewise do not exhibit a constant release profile for the fragrances present over their use time.

The publication WO 85/00981 A1 provides a process for impregnating plastics particles with fragrances at low temperatures. One possible use of the particles is for deodorization in machine dishwashers. These particles are said to be effective for between ten and twenty wash cycles.

US 4 995 556 A discloses a unitized deodorizer for placement in restricted locations where odor control is desirable, which includes a portion of conventional sodium bicarbonate (NaHCO3) or baking soda which is provided with or without a container.

The prior art also discloses deodorants for use in laundry dryers; for instance, the publication US 6,235,705 B1 describes a product for fragrancing in laundry dryers which consists of fragrance-containing plastics pearls in a mesh bag. The pearls are fragranced during their production at elevated temperature.

It is an object of the invention to improve a fragrance release system as per the preamble of claim 1 such that effective fragrance release is ensured even over a prolonged period with minimum space requirement of the container. The dependent claims describe further preferred embodiments.

A fragrance release system according to the present invention comprises a container and a multitude of particles stored in chambers of the container for deodorizing and/or fragrancing open or closed spaces, such as textile washing machines, dryers, especially machine dishwashers. Said particles having a carrier material, which carrier material is preferably polymeric, and particles having at least one fragrance. The container has a plurality of orifices through which emission of the fragrances of the particles from the chambers outward is possible. The chambers comprise at least a first chamber and a second chamber, wherein the volume of the first chamber is larger than the volume of the second chamber.

The inventive fragrance system as a container comprising two chambers.

The volume ratio of the first chamber to the second chamber is between 1,3 and 2,3.

Preferably, the first chamber and the second chamber form, together, a cross section with a convex front wall and a concave back wall. The container has a convex side, which is the front side, and a concave side, which is the back side. The joined chamber cross sectional shape is, when viewed from outside of the chambers, also convex, on the front wall side, and concave, on the back wall side. Thus, the cross section is crescent like.

Preferably, the container is elongated, comprising two extremities, namely an upper extremity and a lower extremity, wherein the lower extremity is opposite to the upper extremity. For the elongated container, it is preferred that the cross-section of the elongation has a crescent-like shape.

It is also preferred, that the elongated container has a mirror symmetry, for its outline, regarding its front view, along an axis connecting the two extremities. For this case it is further preferred that the chamber separation is not mirror symmetric, regarding the front view of the container, along an axis connecting the two extremities.

Preferably, the container of the fragrance release system of the present invention, is in two parts, one part having the back wall and the other part having the front wall. Further preferably wherein the convexity of the convex back wall extends within the concave region formed by the front wall. In a specially preferred embodiment of the invention, the front wall, has a separator separating the first chamber from the second chamber. With this configuration, it is qualitative and cost efficient to fill the chambers of the container with particles and close the two walls (two parts) together, such that the particles are distributed filling the chambers space, during the closing of the parts.

The separator is preferably is elongated and curved along this elongation. The separator is preferably elongated along the separation between the first and the second chambers, thus orthogonal to the distance between the walls forming the first and the second wall. Further preferred is that the elongation of the separator extends between the extremities of the container, and, if it is curved, progresses with at least one deviation from the straight line between the extremities.

In the present invention, it is preferred that the concave back wall curves inwards and has a slot configured to cooperate with the separator when two parts are connected and thus the container is in closed configuration.

It is preferred that, from the front view, the container is asymmetric in regards to its rotation. The axis of rotation is the front view. Rotation asymmetry means than the container has rotation symmetry only at integer multiples of 360°.

For the fragrance release system of the present invention, it is preferred that the container is tapered in the direction of its upper extremity, such that the summed cross section of the chambers increase from the upper extremity towards the lower extremity, preferably at least until half the distance between extremities. This embodiment is especially preferred in combination with the outline mirror symmetry as described above.

Preferably, the upper extremity of the container has on its exterior a fixture means, such as a hook or other securing means such as adhesive surfaces or the like.

The surface area of the particle conglomerate is sufficiently large to ensure effective fragrance release. The particles consist preferably of those materials as described in particular in the claims of the aforementioned patent application, which are explicitly incorporated by reference as a constituent of the disclosure content of this application.

Preference is given to providing that the chambers are filled fully with particles.

The container may in principle be produced in different ways. The container is preferably comprised by two container parts, preferably one part having the back wall and the other part having the front wall. Preferably, the container parts are formed as cooperating parts, which are suitable for connecting to each other, locking themselves together to form a closed container. The two parts of the container may then be produced in a simple manner in an injection molding process.

Preferably, the two edges of the two container parts are configured to cooperate with each other to enable an easy closing of the two container parts, they may further comprise a snap lock system. In order to be able to connect the two container parts together in a very simple manner, preference is given to providing that the part of the container having the back wall or the front wall has a inwardly recessed edge which connects to the inner side of the other part.

In order to ensure substantially full filling of the container with particles in a simple manner in the case of the two-part design of the container, very particular preference is given to providing that a concave back wall curved inwards. In the filling position, the other part having the convex front wall then initially forms the receptacle for the particles. The amount of particles required to fully fill the container is introduced into this container part, so that the fill level remains somewhat below the edge of the container part. Subsequently, the part having the concave back wall is placed on effectively as a lid and snapped in. The middle, concave region displaces the particles outward and upward, and they also get into the regions of the thus formed chamber which have not yet been filled. The dimensioning is such that, before the particles reach the edge, the edge of one container part reaches the edge of the other container part, so that the particles cannot fall out.

The closed space formed when the container is in closed position, defines the total chambers volume in closed configuration. Preferably, the front wall has a separator, such as a wall, separating the first chamber from the second chamber. Further, preferably, the concave back wall has a slot for cooperating with the separator, so that the separator fits into the slot. With this arrangement, it is possible to fill the chambers, and use the concave part of the back wall as two wedges, one for each chamber, to distribute the particles in each chamber when closing the container. It was found that this last configuration provides the easiest way of closing the container while having an optimum distribution of the particles for each chamber.

The term "chamber" as used in the present application refers to the chamber for containing the particles. The specification of a chamber can be used for any of the chambers for containing the particles according to the invention. It is clear to the reader, that the chambers can be identical or different in regard to several aspects, unless explicitly mentioned otherwise. Obviously "a chamber" can be read as "the chamber" when it is needed for explicitly referring to one of the chambers.

A preferred configuration provides that the container of the fragrance release system or a chamber is dimensioned such that the ratio of the total surface area of all particles in the starting state (i.e. before they are heated or softened for the first time) to the total surface area of the chamber is between 1:0.35 and 1:0.36. Such an optimal ratio can be achieved by the selected container shape. Accordingly, for example in the case of a spherical container, only a ratio of 1:0.227 is achievable which, as has been shown, is insufficient to ensure effective fragrance release with comparable total volume of the particles.

In addition, the container is preferably configured such that the layer thickness of the particles in a chamber filled virtually fully with particles is between 10 and 15 mm and the total volume of the chamber is preferably from 10 to 500 ml, preferably about 40 ml. The volume is dependent upon the intended use of the fragrance release system.

The container of the present invention comprises at least a first chamber and a second chamber, each storing a multitude of particles. The volume of the first chamber is larger than the volume of the second chamber.

In order to be able to mount the fragrance release system in a simple manner, for example in a machine dishwasher, the container has on its exterior a hanging device with which the fragrance release system can be hung preferably in the upper carriage of a machine dishwasher. Alternatives are securing means such as adhesive surfaces, with which the device can be fixed to walls of machine.

In addition to the orifices for emitting the fragrances, the container preferably has a multitude of orifices in the region of the back wall, through which, for example, a certain amount of moisture can get into the container interior and out of it during the wash cycle within a machine dishwasher, which can lead to improved fragrance release. While the orifices in the front wall serve primarily for the emission of the fragrance from the particles outward, for example into the interior of a machine dishwasher, the preferably orifices on the back wall of the container are provided to enable water to run in the course of a washing operation, in order to improve the fragrance release.

In the embodiment in which the container is elongated, the orifices in the front wall and preferably also the orifices on the back wall, are distributed on a curve going from one side of the elongation to the other side. There is preferably one of such distribution curves for each chamber. This configuration is especially advantageous when the container is container is tapered in the direction of its upper extremity. Because the cross section is lower towards the upper extremity, it has less contact with water, because the water tends to accumulate in the bottom by flowing from upper part.

It is preferred to have to orifices at the lower extremity of the container for draining remaining water in the container. That enables a longer life for the system as it has less contact with water when not in use.

In one preferred embodiment the two chambers have particles comprise different fragrance. Alternatively or in addition, the two chambers contain particles of a different color and/or shape, preferably a different color.

The combination of particles with different color in the two chambers is especially interesting. The colors can be selected to wear out differently, for example one color gets easily bleached while the other is very stable. The contrast of the colors between the chambers thus gives an indication of the usage of the system. A user can then use this information to discard the system and use a new one. In one special further development, the both chambers are filled with particles having the same fragrance, therefore facilitating the selection of the fragrance, since the usage information is given by the color.

In the context of the present application, the term "polymers" embraces addition polymers, polyadducts and polycondensates.

In this application, addition polymers refer to those high molecular weight compounds which are formed by a chain growth mechanism. Preferred addition polymers in the context of the present application are polyethylene, polypropylene, poly-1-butene, poly-4-methyl-1-pentene, polyvinyl chloride, polyvinylidene chloride, polyacrylonitrile and/or polystyrene.

Polyadducts are formed by polyaddition, i.e. poly reactions, in which repeating and mutually independent linkage reactions of bis- or polyfunctional reactants (monomers) result via reactive oligomers finally in polymers. Preferred polyadducts are polyurethanes.

Like the polyadducts, polycondensates are formed as a result of repeating and mutually independent linkage reactions of discrete oligomers and monomers, except that, in contrast to polyaddition, there is simultaneous elimination of low molecular weight compounds. Preferred polycondensates in the context of the present invention are polyamides, polycarbonates and polyesters.

In summary, the containers for accommodating the fragrance-containing particles comprise at least a proportion of polyethylene, polypropylene, polyethylene/polypropylene copolymers, polyether/polyamide block copolymers, styrene/butadiene (block) copolymers, styrene/isoprene (block) copolymers, styrene/ethylene/butylene copolymers, acrylonitrile/butadiene/styrene copolymers, acrylonitrile/butadiene copolymers, polyether esters, polyisobutene, polyisoprene, ethylene/ethyl acrylate copolymers, polyamides, polycarbonate, polyester, polyacrylonitrile, polymethyl methacrylate or polyurethanes.

Polymers are notable for particular versatility also with regard to their processibility. It is equally possible to process plastics with shaping by extrusion or injection-molding processes as it is by thermoforming processes.

In the course of thermoforming (warm shaping), a preheated plastics plaque or film is introduced between the two parts of the tool, the positive and the negative, which are then compressed together, which results in the plastics part obtaining its shape. What is known as cold shaping proceeds similarly; here, however, the plaque or film to be shaped is not preheated. When there is no negative tool, this is referred to as deep drawing. In the context of the present invention, the abovementioned containers may be produced by all processes known to those skilled in the art, in particular by extrusion, injection molding, thermoforming or blow molding. In any case, the containers have to enable the escape of fragrance and optional other active ingredients, for which purpose both the use of containers equipped with orifices and the at least proportional use of permeable container materials as permeable membranes is suitable. If a separator is used, then it cannot be formed in a single thermoforming process with the container part, but it has to be added separately to the container part. Alternatively a container part can be subdivided in smaller parts, wherein each can be thermoformed, however that may increase complexity.

In the context of the present application, "textile material" refers to those substances which can be processed to textile fabrics. In addition to the synthetic polymers such as nylon, polyester, polyacrylic or polyolefins, the preferred textile materials also include the vegetable materials such as cotton or other cellulosic materials.

Depending upon the type of production process selected and/or a selected coating, the surface of the solid particles may have unevenness.

Further suitable container shapes are bags, nets, sacks, pouches or sachets, which can be produced, for example, from films, nonwovens, wovens or loop-drawn knits, and their pore or mesh width, as in the case of the aforementioned injection moldings, blow moldings or thermoformings, has to be lower than the diameter of the particles in order to prevent trickling out.

After the introduction of the fragrance-containing particles, the aforementioned containers have to closed. They may be closed by clipping them together. Alternatively, they may be sealed by sewing, fusion bonding or adhesion bonding.

Preference is given to providing that the chamber is filled fully with particles.

It has been found that the further object is achieved by fragrance release systems which have certain polymeric carrier materials. The present application therefore also provides a fragrance release system comprising a vessel and particles for deodorizing and fragrancing spaces, which comprises at least one polymeric carrier material having a melting or softening point between 30 and 150°C and at least one fragrance. In the case of this fragrance release system, particles are used whose polymeric carrier material has a softening or melting point between 30° and 150°C and even more preferably between 75° and 80°C. Such a fragrance release system has an optimized fragrance release profile which is based on a change in the ratios of surface area to internal volume of the particles present in the fragrance release system. In a brand new, unused system, these particles are present as singular individual particles having a large particle surface area. However, the composition of the particles is selected such that, in the event of thermal stress on these particles, the surface of the polymeric carrier material of these particles softens or melts and individual particles adhere together with reduction of the overall surface area. The magnitude of the melting or softening point of the polymeric carrier materials is determined by the field of use. For example, maximum temperatures between 65° and 75°C occur during machine dishwashing, especially in the rinse cycle. The container of this fragrance release system consists preferably of a water-insoluble organic or inorganic material, such as plastic, ceramic, glass, metal or textiles.

The improved fragrance release of inventive fragrance release systems is based on a change in the ratio of surface area to internal volume of the particles present in the fragrance release system. In a brand new, unused system, these particles are present as singular particles with large particle surface area. However, the composition of the particles is selected such that, in the event of thermal stress on these particles, the surface of the polymeric support material of these particles is softened or melted and individual particles adhere together with reduction of the overall surface area. The magnitude of the melting or softening point of the polymeric support materials is accordingly determined by the field of use of inventive compositions. For example, maximum temperatures between 65 and 75°C occur during machine dishwashing, especially in the rinse cycle. When inventive compositions serve to fragrance spaces in buildings or vehicles, for example as an attachment for heaters, the maximum temperatures attained there are generally in the range from 70 to 90°C. In any case, the melting or softening points of the particles should be above the ambient temperature customary in the course of the transport and storage thereof and below the decomposition temperatures of the fragrances present.

Suitable polymeric carrier materials for the fragrance-containing particles are generally all polymers or polymer mixtures which satisfy the abovementioned criteria with regard to the melting or softening temperature. Fragrance release systems preferred in the context of the present application are characterized in that the polymeric carrier material comprises at least one substance from the group consisting of ethylene/vinyl acetate copolymers, low- or high-density polyethylene (LDPE, HDPE) or mixtures thereof, polypropylene, polyethylene/polypropylene copolymers, polyether/polyamide block copolymers, styrene/butadiene (block) copolymers, styrene/isoprene (block) copolymers, styrene/ethylene/butylene copolymers, acrylonitrile/butadiene/styrene copolymers, acrylonitrile/butadiene copolymers, polyether esters, polyisobutene, polyisoprene, ethylene/ethyl acrylate copolymers, polyamides, polycarbonate, polyester, polyacrylonitrile, polymethyl methacrylate, polyurethanes, polyvinyl alcohols.

Polyethylene (PE) is a collective term for the polymers which belong to the polyolefins and have moieties of the CH₂-CH₂ type as a characteristic base unit of the polymer chain. Polyethylenes are prepared generally by addition polymerization of ethylene by two fundamentally different methods, the high-pressure and the low-pressure process. The resulting products are correspondingly frequently referred to as high-pressure polyethylenes and low-pressure polyethylenes respectively; they differ mainly with regard to their degree of branching and, associated with this, in their degree of crystallinity and their density. Both processes may be carried out as a solution polymerization, emulsion polymerization or gas phase polymerization.

In the high-pressure process, branched polyethylenes of low density (approx. 0.915-0.935 g/cm³), and degrees of crystallinity of approx. 40-50% are achieved, which are referred to as LDPE (low-density polyethylene) types. Products of higher molar mass and, as a result of this, improved strength and stretchability have the abbreviation HMW-LDPE (HMW = high molecular weight). Copolymerization of ethylene with longer-chain olefins, especially with butene and octene, allows the marked degree of branching of the polyethylenes prepared in the high-pressure process to be reduced; the copolymers have the abbreviation LLD-PE (linear low-density polyethylene).

The macromolecules of the polyethylenes from low-pressure processes are substantially linear and unbranched. These polyethylenes, abbreviated to HDPE (high-density polyethylene), have degrees of crystallinity of 60-80% and a density of approx. 0.94-0.965 g/cm³. They are supplied as products having high and ultrahigh molar mass (approx. 200 000-5 000 000 g/mol and 3 000 000-6 000 000 g/mol respectively), under the abbreviation HD-HMW-PE and UHMW-HD-PE respectively. Products of medium density (MDPE) composed of mixtures of polyethylenes of low and high density are also commercially available. Linear polyethylenes having densities of < 0.918 g/cm³ (VLD-PE, very low-density polyethylene) are only slowly gaining importance on the market.

Polyethylenes have a very low water vapor permeability; the diffusion of gases, and also of aromas and ethereal substances, through polyethylene is relatively high. The mechanical properties are greatly dependent upon molecular size and structure of the polyethylenes. Generally, degree of crystallinity and density of polyethylenes increase with decreasing degree of branching and with shortening of the side chains. Shear modulus, hardness, stretching limit and melting range increase with density; shock resistance, transparency, swellability and solubility decrease. At the same density, tensile strain at break, elongation, shock resistance, impact strength and sustained use strength increase with rising molar mass of the polyethylenes. Depending on the procedure in the polymerization, it is possible to obtain products having paraffin wax-like properties (MR about 2000) and products of maximum toughness (MR above 1 million).

The polyethylene types may be processed by all methods customary for thermoplastics.

Polypropylene (PP) is the name for thermoplastic polymers of propylene with the general formula: - (CH₂-CH[CH₃])ₙ-

The basis of polypropylene preparation was the development of the process for the stereospecific polymerization of propylene in the gas phase or in suspension by Natta. This is initiated by Ziegler-Natta catalysts, but to an increasing degree also by metallocene catalysts, and leads either to highly crystalline isotactic or to less crystalline syndiotactic or to amorphous atactic polypropylenes.

Polypropylene features high hardness, rebound resilience, stiffness and heat resistance. It is possible to briefly heat objects made of propylene even up to 140°C. At temperatures below 0°C, a certain embrittlement of the polypropylenes occurs, but can be shifted to substantially lower temperature ranges by copolymerization of the propylene with ethylene (EPM, EPDM). Generally, the impact strength of polypropylene can be improved by modification with elastomers. As in the case of all polyolefins, the chemical resistance is good. An improvement in the mechanical properties of the polypropylenes is achieved by reinforcing with talc, chalk, wood meal or glass fibers. Polypropylenes are oxidation- and light-sensitive to an even greater degree than PE, which is why it is necessary to add stabilizers (antioxidants, light stabilizers, UV absorbers).

Polyethers is an umbrella term in the field of macromolecular chemistry for polymers whose organic repeating units are held together by ether functionalities (C-O-C). According to this definition, a multitude of structurally very different polymers belongs to the polyethers, for example the polyalkylene glycols (polyethylene glycols, polypropylene glycols and polyepichlorohydrins) as polymers of 1,2-epoxides, epoxy resins, polytetrahydrofurans (polytetramethylene glycols), polyoxetanes, polyphenylene ethers (see polyaryl ethers) or polyether ether ketones (see polyether ketones). The polyethers do not include polymers having pendent ether groups, including the cellulose ethers, starch ethers and vinyl ether polymers.

The group of the polyethers also includes functionalized polyethers, i.e. compounds having a polyether structure which also bear, attached pendent to their main chains, other functional groups, for example carboxyl, epoxy, allyl or amino groups, etc. Block copolymers of polyethers and polyamides (known as polyether amides or polyether block amides, PEBA) have a variety of possible uses.

Polyamides (PA) refer to polymers whose basic units are held together by amide bonds (-NH-CO-). Naturally occurring polyamides are peptides, polypeptides and proteins (for example albumin, wool, silk). Apart from a few exceptions, the synthetic polyamides are thermoplastic, catenated polymers, some of which have gained great industrial significance as synthetic fibers and materials. According to the chemical structure, what are known as the homopolyamides can be divided into two groups, the aminocarboxylic acid types (AC) and the diamine-dicarboxylic acid types (AA-CC; A denotes amino groups and C carboxyl groups). The former are prepared from only a single monomer by, for example, polycondensation of an ω-aminocarboxylic acid (1) (polyamino acids) or by ring-opening polymerization of cyclic amides (lactams) (2).

In addition to the homopolyamides, some copolyamides have also gained significance. It is customary to qualitatively and quantitatively specify the composition, for example PA 66/6 (80:20) for polyamides prepared from 1,6-hexanediamine, adipic acid and ε-caprolactam in a molar ratio of 80:80:20.

Owing to their special properties, polyamides which contain exclusively aromatic radicals (for example those made from p-phenylenediamine and terephthalic acid) are embraced under the generic name of aramids or polyaramids (for example Nomex^{®}).

The most frequently used polyamide types (in particular PA 6 and PA 66) consist of unbranched chains with average molar masses of from 15 000 to 50 000 g/mol. They are semicrystalline in the solid state and have degress of crystallization of 30-60%. An exception is that of polyamides made from units having side chains or copolyamides made from highly differing components, which are substantially amorphous. In contrast to the generally milky, opaque semicrystalline polyamides, these are almost glass-clear. The softening temperature of the most commonly used homopolyamides is between 200 and 260°C (PA 6: 215-220°C, PA 66: 255-260°C).

Polyesters is the collective term for polymers whose basic units are held together by ester bonds (-CO-O-). According to their chemical structure, what are known as the homopolyesters can be divided into two groups, the hydroxycarboxylic acid types (AB polyesters) and the dihydroxydicarboxylic acid types (AA-BB polyesters). The former are prepared from only a single monomer by, for example, polycondensation of an ω-hydroxycarboxylic acid 1 or by ring-opening polymerization of cyclic esters (lactones) 2.

Branched and crosslinked polyesters are obtained in the polycondensation of tri- or polyhydric alcohols with polyfunctional carboxylic acids. The polyesters generally also include the polycarbonates (polyesters of carbonic acid).

AB-type polyesters (I) include polyglycolic acids, polylactic acids, polyhydroxybutyric acid [poly(3-hydroxybutyric acid)], poly(ε-caprolactone)s and polyhydroxybenzoic acids.

Purely aliphatic AA-BB-type polyesters (II) are polycondensates of aliphatic diols and dicarboxylic acids which can be used, among other uses, as products having terminal hydroxyl groups (as polydiols) for the preparation of polyester polyurethanes [for example polytetramethylene adipate]. In quantitative terms, the greatest industrial significance is possessed by AA-BB-type polyesters made from aliphatic diols and aromatic dicarboxylic acids, in particular the polyalkylene terephthalates, with polyethylene terephthalate (PET), polybutylene terephthalate (PBT) and poly(1,4-cyclohexanedimethylene terephthalate)s (PCDT) as the most important representatives. The properties of these types of polyesters can be varied widely by also using other aromatic dicarboxylic acids (e.g. isophthalic acid) or by using diol mixtures in the polycondensation, and they can be adapted to different fields of use.

Purely aromatic polyesters are the polyarylates, which include poly(4-hydroxybenzoic acid). In addition to the saturated polyesters mentioned hitherto, it is also possible to prepare unsaturated polyesters from unsaturated dicarboxylic acids, which have gained industrial significance as polyester resins, in particular as unsaturated polyester resins (UP resins).

Polyesters are generally thermoplastics. Products based on aromatic dicarboxylic acids have marked materials character. The purely aromatic polyarylates feature high thermal stability.

Polyurethanes (PUR) denote polymers in whose macromolecules the repeating units are joined by urethane moieties -NH-CO-O-. Polyurethanes are obtained generally by polyaddition of dihydric or higher polyhydric alcohols and isocyanates.

Depending on the selection and stoichiometric ratio of the starting materials, polyurethanes are thus formed which have very different mechanical properties and can be used as constituents of adhesives and coatings (polyurethane resins), as ionomers, as a thermoplastic material for bearing parts, castors, tires, rolls, and as more or less hard elastomers in fiber form (elastomeric fibers, abbreviated to PUE for these elastan or spandex fibers) or as polyether or polyesterurethane rubber (EU and AU respectively).

Polyurethane foams are formed in the polyaddition when water and/or carboxylic acids are present because these react with the isocyanates with the elimination of carbon dioxide which has a swelling and foam-forming action. The use of polyalkylene glycol ethers as diols and water as a reaction component leads to flexible polyurethane foams; the use of polyols and propellant gases from CFCs (particularly R11) affords rigid polyurethane foams and structural or integral foams. Examples of assistants additionally required here are catalysts, emulsifiers, foam stabilizers (particularly polysiloxane-polyether copolymers), pigments, aging inhibitors and flame retardants. For the production of objects made of polyurethane foam, even those having a complicated shape, what is known as the RIM technique (reaction injection molding) was developed in the 1970s. The RIM process is based on rapid metering and mixing of the components, injection of the reactive mixture into the mold and rapid curing; the cycle time is only a few minutes. By means of the RIM technique, objects including automotive bodywork parts, shoe soles, window profiles and television casings are obtained.

In a particularly preferred embodiment of the present invention, the polymeric carrier material of the particles consists at least proportionally of ethylene/vinyl acetate copolymer. The present application therefore further preferably provides a fragrance release system, characterized in that the polymeric carrier material contains at least 10% by weight, preferably at least 30% by weight, more preferably at least 70% by weight, of ethylene/vinyl acetate copolymer, and is preferably produced fully from ethylene/vinyl acetate copolymer.

Ethylene/vinyl acetate copolymers is the term for copolymers made of ethylene and vinyl acetate. This polymer is in principle prepared in a process comparable to the preparation of polyethylene of low density (LDPE; low-density polyethylene). With an increasing proportion of vinyl acetate, the crystallinity of the polyethylene is disrupted and the melting and softening points and the hardness of the resulting products are lowered in this way. The vinyl acetate additionally makes the copolymer more polar and thus improves its adhesion to polar substrates.

The above-described ethylene/vinyl acetate copolymers are commercially widely available, for example under the trademark Elvax^{®} (Dupont). Particularly suitable polyvinyl alcohols in the context of the present invention are, for example, Elvax^{®} 265. Other suitable particles are described in the prior art, such as EP1526876 A1.

Some particularly suitable copolymers and their physical properties can be taken from the table below:
In the context of the present invention, especially in the field of fragrancing the interiors of machine dishwashers, particular preference is given to fragrance release systems in which the polymeric carrier material used is ethylene/vinyl acetate copolymer and this copolymer contains from 5 to 50% by weight of vinyl acetate, preferably from 10 to 40% by weight of vinyl acetate and in particular from 20 to 30% by weight of vinyl acetate, based in each case on the total weight of the copolymer.

Inventive fragrance release systems comprise the polymeric carrier materials in the form of particles. The three-dimensional shape of these particles is restricted merely by the technical possibilities in their production. Possible three-dimensional shapes are all embodiments which can be handled viably, i.e., for example, cubes, cuboids and corresponding three-dimensional elements having flat side surfaces, and also in particular cylindrical embodiments with circular or oval cross section. This last embodiment embraces tablet-shaped particles up to compact cylinder sections having a ratio of height to diameter above 1. Further possible three-dimensional shapes are spheres, hemispheres or "stretched spheres" in the form of ellipsoidal capsules, as are regular polyhedra, for example tetrahedra, hexahedra, octahedra, dodecahedra, icosahedra. Also conceivable are star-shaped embodiments with three, four, five, six or more points or fully irregular bodies which can be configured, for example, in a motif. Suitable motifs, depending upon the field of use of the inventive compositions are, for example, animal figures such as dogs, horses or birds, floral motifs or the illustration of fruits. However, the motif-type embodiment may also relate to inanimate objects such as vehicles, tools, household objects or clothing. The surface of the solid particles may have unevenness depending upon the type of production process selected and/or a selected coating. Owing to the numerous possible embodiments of the particles, the inventive compositions are notable for advantages not only in their production. Owing to the numerous embodiment forms, the fragrance-containing particles are additionally clearly perceptible visually to the consumer and enable, by the selective spatial configuration of these particles, a visualization, particularly advantageous for product acceptance, of the fragrances present in the inventive compositions or further active substances optionally present in these compositions. For instance, the visually perceptible multiphasicity of these compositions may illustrate, for example, the differing function of individual active substances (for example cleaning and additional functions such as glass protection, silver protection, etc.).

In the context of the present application, particles have a solid consistency at room temperature, i.e. dimensionally stable and not free-flowing. Preferred particles have an average diameter of from 0.5 to 20 mm, preferably of from 1 to 10 mm and in particular of from 3 to 6 mm.

The polymeric carrier materials can be formulated to give the above-described particles by all processes known to those skilled in the art for the processing of these substances. Preference is given in the context of the present invention to extrusion, injection molding and spraying to give polymer granules.

In addition to a vessel, inventive fragrance release systems also comprise fragrance-containing particles based on polymeric carrier materials, the proportion by weight of the fragrance(s), based on the total weight of the particles, being preferably from 1 to 70% by weight, preferably from 10 to 60% by weight, more preferably from 20 to 50% by weight, in particular from 30 to 40% by weight.

In the context of the present invention, the perfume oils or fragrances used may be individual odorant compounds, for example the synthetic products of the ester, ether, aldehyde, ketone, alcohol and hydrocarbon type. Odorant compounds of the ester type are, for example, benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethyl methylphenylglycinate, allyl cyclohexylpropionate, styrallyl propionate and benzyl salicylate. The ethers include, for example, benzyl ethyl ether; the aldehydes include, for example, the linear alkanals having from 8 to 18 carbon atoms, citral, citronellal, citronellyloxyacetaldehyde, cyclamen aldehyde, hydroxycitronellal, lilial and bourgeonal; the ketones include, for example, the ionones, α-isomethylionone and methyl cedryl ketone; the alcohols include anethol, citronellol, eugenol, geraniol, linalool, phenylethyl alcohol and terpineol; the hydrocarbons include primarily the terpenes, such as limonene and pinene. However, preference is given to using mixtures of different odorants which together generate a pleasing fragrance note. Such perfume oils may also contain natural odorant mixtures, as obtainable from vegetable sources, e.g. pine oil, citrus oil, jasmine oil, patchouli oil, rose oil and ylang-ylang oil. Likewise suitable are muscatel, sage oil, chamomile oil, oil of cloves, melissa oil, mint oil, cinnamon leaf oil, lime blossom oil, juniperberry oil, vetiver oil, olibanum oil, galbanum oil and labdanum oil, and orange blossom oil, neroliol, orange peel oil and sandalwood oil.

The general description of the perfumes which can be used (see above) is a general representation of the different classes of odorant substances. In order to be perceptible, an odorant must be volatile, for which an important role is played not only by the nature of the functional groups and by the structure of the chemical compound but also by the molar mass. Thus, the majority of odorants have molar masses of up to about 200 daltons, while molar masses of 300 daltons or more tend to be an exception. On the basis of the different volatility of odorants there is a change in the odor of a perfume or fragrance composed of two or more odorants during its evaporation, and the perceived odors are divided into top note, middle note or body, and end note or dryout. Since the perception of odor is to a large extent also based on the odor intensity, the top note of a perfume or fragrance mixture does not consist only of volatile compounds, whereas the base note consists for the most part of less volatile odorants, i.e., odorants which adhere firmly. In the composition of perfumes it is possible for more volatile odorants, for example, to be bound to certain fixatives, which prevent them from evaporating too rapidly. The subsequent classification of the odorants into "more volatile" and "firmly adhering" odorants, therefore, states nothing about the perceived odor and about whether the odorant in question is perceived as a top note or as a middle note.

An appropriate selection of the fragrances and perfume oils mentioned allows the adjustment of both, the product scent emitted directly on opening, and the product scent emitted during use, for example when used in a machine dishwasher. These perceived fragrances may of course be the same but they may also be different. It is advantageous to use more firmly adhering odorants for the latter perceived odor, while more volatile odorants can also be used to fragrance the product. Examples of firmly adhering odorants which can be used in the context of the present invention are the essential oils such as angelica root oil, anise oil, arnica blossom oil, basil oil, bay oil, bergamot oil, champaca blossom oil, noble fir oil, noble fir cone oil, elemi oil, eucalyptus oil, fennel oil, spruce needle oil, galbanum oil, geranium oil, ginger grass oil, guaiacwood oil, gurjun balsam oil, helichrysum oil, ho oil, ginger oil, iris oil, cajeput oil, calamus oil, chamomile oil, camphor oil, canaga oil, cardamom oil, cassia oil, pine needle oil, copaiva balsam oil, coriander oil, spearmint oil, caraway oil, cumin oil, lavender oil, lemon grass oil, lime oil, mandarin oil, melissa oil, musk seed oil, myrrh oil, oil of cloves, neroli oil, niaouli oil, olibanum oil, orange oil, origanum oil, palmarosa oil, patchouli oil, Peru balsam oil, petitgrain oil, pepper oil, peppermint oil, pimento oil, pine oil, rose oil, rosemary oil, sandalwood oil, celery oil, spike oil, star anise oil, turpentine oil, thuja oil, thyme oil, verbena oil, vetiver oil, juniperberry oil, wormwood oil, wintergreen oil, ylang-ylang oil, hyssop oil, cinnamon oil, cinnamon leaf oil, citronellol, lemon oil and cypress oil. However, the higher-boiling or solid odorants of natural or synthetic origin may also be used in the context of the present invention as firmly adhering odorants or odorant mixtures, i.e. fragrances. These compounds include the following compounds and mixtures thereof: ambrettolide, α-amylcinnamaldehyde, anethole, anisaldehyde, anisyl alcohol, anisole, methyl anthranilate, acetophenone, benzylacetone, benzaldehyde, ethyl benzoate, benzophenone, benzyl alcohol, benzyl acetate, benzyl benzoate, benzyl formate, benzyl valerate, borneol, bornyl acetate, α-bromostyrene, n-decyl aldehyde, n-dodecylaldehyde, eugenol, eugenol methyl ether, eucalyptol, farnesol, fenchone, fenchyl acetate, geranyl acetate, geranyl formate, heliotropin, methyl heptynecarboxylate, heptaldehyde, hydroquinone dimethyl ether, hydroxycinnamaldehyde, hydroxycinnamyl alcohol, indole, irone, isoeugenol, isoeugenol methyl ether, isosafrol, jasmone, camphor, carvacrol, carvone, p-cresol methyl ether, coumarin, p-methoxyacetophenone, methyl n-amyl ketone, methyl methylanthranilate, p-methylacetophenone, methylchavicol, p-methylquinoline, methyl β-naphthyl ketone, methyl-n-nonylacetaldehyde, methyl n-nonyl ketone, muscone, β-naphthol ethyl ether, β-naphthol methyl ether, nerol, nitrobenzene, n-nonylaldehyde, nonyl alcohol, n-octylaldehyde, p-oxyacetophenone, pentadecanolide, β-phenylethyl alcohol, phenylacetaldehyde dimethyl acetal, phenylacetic acid, pulegone, safrol, isoamyl salicylate, methyl salicylate, hexyl salicylate, cyclohexyl salicylate, santalol, skatole, terpineol, thymene, thymol, γ-undecalactone, vanillin, veratrum aldehyde, cinnamaldehyde, cinnamyl alcohol, cinnamic acid, ethyl cinnamate, benzyl cinnamate. The more volatile odorants include in particular the lower-boiling odorants of natural or synthetic origin, which may be used alone or in mixtures. Examples of more volatile odorants are alkyl isothiocyanates (alkyl mustard oils), butanedione, limonene, linalool, linalyl acetate and linalyl propionate, menthol, menthone, methyl-n-heptenone, phellandrene, phenylacetaldehyde, terpinyl acetate, citral, citronellal.

Preference is given to loading the plastics particles with the selected fragrance at a temperature of from 15 to 30°C, preferably of from 20 to 25°C. To this end, the particles are admixed with the appropriate amount of the fragrance and mixed. In any case, the temperature should, though, be below the melting or decomposition temperature of the plastic and also below the flashpoint of the perfume oil. The fragrance is absorbed by the polymeric carrier material or by further perfume carrier materials present in the particles primarily by adhesion, diffusion and/or capillary forces, and the particles may swell slightly in the course of this operation.

As mentioned above, inventive compositions may comprise, apart from the constituents needed for fragrancing and deodorization, further active substances. It is accordingly possible to distinguish, from the compositions which serve exclusively for fragrancing, further product groups which, in addition to the aforementioned inventive constituents, comprise further preferred substances.

A first of these optionally usable preferred substances is the dyes. Suitable for this purpose are generally all dyes which are known by those skilled in the art to be suitable for coloring plastics and to be soluble in perfume oils. Preference is given to selecting the dye according to the fragrance used; for example, particles having a lemon fragrance preferably have a yellow color, while preference is given to a green color for particles having an apple or herb fragrance. Preferred dyes have high storage stability and insensitivity toward the remaining ingredients of the compositions and to light. When the inventive compositions are used in connection with textile cleaning or dishwashing, the dyes used should not have any marked substantivity toward textile fibers, glass, plasticware or ceramics, in order not to stain them.

Suitable dyes and dye mixtures are commercially available under various trade names and are supplied by firms including BASF AG, Ludwigshafen, Bayer AG, Leverkusen, Clariant GmbH, DyStar Textilfarben GmbH & Co. Deutschland KG, Les Colorants Wackherr SA and Ciba Specialty Chemicals. The suitable fat-soluble dyes and dye mixtures include, for example, Solvent Blue 35, Solvent Green 7, Solvent Orange 1 (Orange au Gras-W-2201), Sandoplast Blau 2B, Fettgelb 3G, Iragon^{®} Red SRE 122, Iragon^{®} Green SGR 3, Solvent Yellow 33 and Solvent Yellow 16, but other dyes may also be present.

In a preferred embodiment, the dye, in addition to its esthetic effect, additionally has an indicator function. This indicates to the consumer the actual consumption level of the deodorant, so that he/she obtains, in addition to the absence of fragrance impression which may, for example, be based on an accustoming effect on the part of the user, a further reliable indication as to when the deodorant should be replaced by a new one.

The indicator effect may be achieved in various ways: one way is to use a dye which escapes from the particles in the course of the use time. This may be brought about, for example, by the ingredients present in the dishwasher detergent. To this end, a dye has to be used which adheres well to the particles and only slowly diffuses out of them, in order to ensure that the discoloration is not complete too early, i.e. when the fragrance has not yet been consumed. Another way is that a color change is brought about by a chemical reaction or thermal decomposition.

Further preferred constituents of inventive compositions are substances such as active antimicrobial ingredients, germicides, fungicides, antioxidants or corrosion inhibitors, with the aid of which additional uses, for example disinfection or corrosion protection, can be realized.

For the control of microorganisms, the inventive compositions may comprise active antimicrobial ingredients. Depending on the antimicrobial spectrum and mechanism of action, a distinction is drawn between bacteriostats and bacteriocides, fungistats and fungicides, etc. Important substances from these groups are, for example, benzalkonium chlorides, alkylarylsulfonates, halophenols and phenylmercuric acetate.

In order to prevent undesired changes, caused by the action of oxygen and other oxidative processes, to the inventive compositions or to the treated, for example textiles, the compositions may comprise antioxidants. This compound class includes, for example, substituted phenols, hydroquinones, pyrocatechols, and aromatic amines, and also organic sulfides, polysulfides, dithiocarbamates, phosphites and phosphonates.

When the inventive compositions are used in machine dishwashers, these compositions may comprise corrosion inhibitors to protect the ware or the machine, and particularly silver protectants have special significance in the field of machine dishwashing. It is possible to use the known substances of the prior art. Generally, it is possible in particular to use silver protectants selected from the group of the triazoles, the benzotriazoles, the bisbenzotriazoles, the aminotriazoles, the alkylaminotriazoles and the transition metal salts or complexes. Particular preference is given to using benzotriazole and/or alkylaminotriazole. Frequently also found in cleaning formulations are active chlorine-containing agents which can significantly reduce the corrosion of the silver surface. In chlorine-free detergents, particularly oxygen- and nitrogen-containing organic redox-active compounds, such as di- and trihydric phenols, for example hydroquinone, pyrocatechol, hydroxyhydroquinone, gallic acid, phloroglucine, pyrogallol and derivatives of these classes of compound, are used. Salt- and complex-type inorganic compounds, such as salts of the metals Mn, Ti, Zr, Hf, V, Co and Ce, also frequently find use. Preference is given in this context to the transition metal salts which are selected from the group of manganese and/or cobalt salts and/or complexes, more preferably cobalt(ammine) complexes, cobalt(acetate) complexes, cobalt(carbonyl) complexes, the chlorides of cobalt or manganese, and manganese sulfate. Zinc compounds may likewise be used to prevent corrosion on the ware.

Instead of or in addition to the above-described silver protectants, for example the benzotriazoles, it is possible to use redox-active substances in the inventive compositions. These substances are preferably inorganic redox-active substances from the group of the manganese, titanium, zirconium, hafnium, vanadium, cobalt and cerium salts and/or complexes, the metals preferably being in one of the oxidation states II, III, IV, V or VI.

The metal salts or metal complexes used should be at least partially soluble in water. The counterions suitable for the salt formation include all customary singly, doubly or triply negatively charged inorganic anions, for example oxide, sulfate, nitrate, fluoride, but also organic anions, for example stearate.

Metal complexes in the context of the invention are compounds which consist of a central atom and one or more ligands, and optionally additionally one or more of the abovementioned anions. The central atom is one of the abovementioned metals in one of the abovementioned oxidation states. The ligands are neutral molecules or anions which are mono- or polydentate; the term "ligands" in the context of the invention is explained in more detail, for example, in "Römpp Chemie Lexikon, Georg Thieme Verlag, Stuttgart/New York, 9th edition, 1990, page 2507". When the charge of the central atom and the charge of the ligand(s) within a metal complex do not add up to zero, depending on whether there is a cationic or an anionic charge excess, either one or more of the abovementioned anions or one or more cations, for example sodium, potassium, ammonium ions, ensure that the charge balances. Suitable complexing agents are, for example, citrate, acetyl acetonate or 1-hydroxyethane-1,1-diphosphonate.

The definition of "oxidation state" customary in chemistry is reproduced, for example, in "Römpp Chemie Lexikon, Georg Thieme Verlag, Stuttgart/New York, 9th edition, 1991, page 3168".

Particularly preferred metal salts and/or metal complexes are selected from the group of MnSO₄, Mn(II) citrate, Mn(lI) stearate, Mn(II) acetylacetonate, Mn(II) [1-hydroxyethane-1,1-diphosphonate], V₂O₅, V₂O₄, VO₂, TiOSO₄, K₂TiF₆, K₂ZrF₆, COSO₄, CO(NO₃)₂, Ce(NO₃)₃, and mixtures thereof, so that preferred inventive compositions are characterized in that the metal salts and/or metal complexes are selected from the group of MnSO₄, Mn(II) citrate, Mn(II) stearate, Mn(II) acetylacetonate, Mn(II) [1-hydroxyethane-1,1-diphosphonate], V₂O₅, V₂O₄, VO₂, TiOSO₄, K₂TiF₆, K₂ZrF₆, COSO₄, CO(NO₃)₂, Ce(NO₃)₃.

These metal salts or metal complexes are generally commercial substances which can be used in the inventive compositions for the purposes of silver corrosion protection without prior purification. For example, the mixture of penta- and tetravalent vanadium (V₂O₅, VO₂, V₂O₄) known from the preparation of SO₃ (contact process) is therefore suitable, as is the titanyl sulfate TiOSO₄ which is obtained by diluting a Ti(SO₄)₂ solution.

The metal salts and/or metal complexes mentioned are present in the inventive compositions preferably in an amount of from 0.05 to 6% by weight, preferably from 0.2 to 2.5% by weight, based on the overall composition without the container.

A further important criterion for the assessment of a machine dishwasher detergent is, aside from its cleaning performance, the visual appearance of the dry dishes on completion of cleaning. Any calcium carbonate deposits which arise on dishes or in the interior of the machine might, for example, impair customer satisfaction and thus have a causal influence on the economic success of such a detergent. A further problem which has existed for some time in machine dishwashing is the corrosion of glassware, which can usually manifest itself by the appearance of clouding, smearing and scratches, but also by an iridescence of the glass surface. The observed effects are based essentially on two operations, firstly the exit of alkali metal and alkaline earth metal ions from the glass in conjunction with a hydrolysis of the silicate network, and secondly in a deposition of silicatic compounds on the glass surface.

The problems mentioned can be solved using the inventive compositions when, in addition to the aforementioned obligatory and any optional ingredients, certain glass corrosion inhibitors are incorporated into the compositions. Preferred inventive compositions therefore additionally comprise one or more magnesium and/or zinc salts and/or magnesium and/or zinc complexes.

A preferred class of compounds which can be added to the inventive compositions to prevent glass corrosion is that of insoluble zinc salts. These can position themselves during the dishwashing operation on the glass surface, where they prevent metal ions from the glass network from going into solution, and also the hydrolysis of the silicates. Additionally, these insoluble zinc salts also prevent the deposition of silicate on the surface of the glass, so that the glass is protected from the consequences outlined above.

In the context of this preferred embodiment, insoluble zinc salts are zinc salts which have a maximum solubility of 10 grams of zinc salt per liter of water at 20°C. Examples of insoluble zinc salts which are particularly preferred in accordance with the invention are zinc silicate, zinc carbonate, zinc oxide, basic zinc carbonate (Zn₂(OH)₂CO₃), zinc hydroxide, zinc oxalate, zinc monophosphate (Zn₃(PO₄)₂), and zinc pyrophosphate (Zn₂(P₂O₇)).

The zinc compounds mentioned are used in the inventive compositions in amounts which bring about a content in the compositions of zinc ions of between 0.02 and 10% by weight, preferably between 0.1 and 5.0% by weight and in particular between 0.2 and 1.0% by weight, based in each case on the composition without the container. The exact content in the compositions of zinc salt or zinc salts is by its nature dependent on the type of the zinc salts - the less soluble the zinc salt used, the higher its concentration in the inventive compositions should be.

A further preferred class of compounds is that of magnesium and/or zinc salt(s) of at least one monomeric and/or polymeric organic acid. These have the effect that, even upon repeated use, the surfaces of glassware are not altered as a result of corrosion, and in particular no clouding, smears or scratches, and also no iridescence of the glass surfaces, are caused.

Even though all magnesium and/or zinc salt(s) of monomeric and/or polymeric organic acids may be present in accordance with the invention in the claimed compositions, preference is given, as described above, to the magnesium and/or zinc salts of monomeric and/or polymeric organic acids from the groups of the unbranched, saturated or unsaturated monocarboxylic acids, the branched, saturated or unsaturated monocarboxylic acids, the saturated and unsaturated dicarboxylic acids, the aromatic mono-, di- and tricarboxylic acids, the sugar acids, the hydroxy acids, the oxo acids, the amino acids and/or the polymeric carboxylic acids. In the context of the present invention, preference is in turn given within these groups to the acids specified below:
The spectrum of the zinc salts, preferred in accordance with the invention, of organic acids, preferably of organic carboxylic acids, ranges from salts which are sparingly soluble or insoluble in water, i.e. have a solubility below 100 mg/l, preferably below 10 mg/l, in particular have zero solubility, to those salts which have a solubility in water above 100 mg/l, preferably above 500 mg/l, more preferably above 1 g/l and in particular above 5 g/l (all solubilities at water temperature 20°C). The first group of zinc salts includes, for example, zinc citrate, zinc oleate and zinc stearate; the group of soluble zinc salts includes, for example, zinc formate, zinc acetate, zinc lactate and zinc gluconate.

In a further preferred embodiment of the present invention, the compositions according to the invention comprise at least one zinc salt, but no magnesium salt of an organic acid, preferably at least one zinc salt of an organic carboxylic acid, more preferably a zinc salt from the group of zinc stearate, zinc oleate, zinc gluconate, zinc acetate, zinc lactate and/or zinc citrate. Preference is also given to zinc ricinoleate, zinc abietate and zinc oxalate.

A composition which is preferred in the context of the present invention contains zinc salt in amounts of from 0.1 to 5% by weight, preferably from 0.2 to 4% by weight and in particular from 0.4 to 3% by weight, or zinc in oxidized form (calculated as Zn²⁺) in amounts of from 0.01 to 1 % by weight, preferably from 0.02 to 0.5% by weight and in particular from 0.04 to 0.2% by weight, based in each case on the composition without the container.

The present application therefore further provides a fragrance release system which comprises further active substances, in particular active substances from the group of the perfume carriers, dyes, active antimicrobial ingredients, germicides, fungicides, antioxidants or corrosion inhibitors.

The invention is illustrated in detail by way of example with reference to the drawing. In the drawing:
Fig.1 shows an inventive fragrance release system in cross section after the introduction of the particles and before the closure of the container,
Fig. 2 shows a fragrance release system as per Figure 1 after the closure of the container,
Fig. 3 shows a cross section through the container of the fragrance release system,

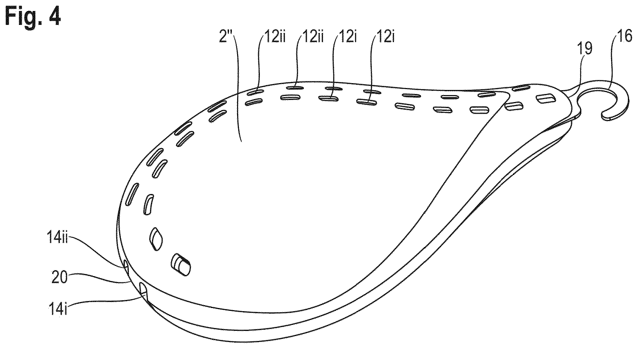

Fig. 4 shows the closed container of the fragrance release system in perspective representation in back view, and

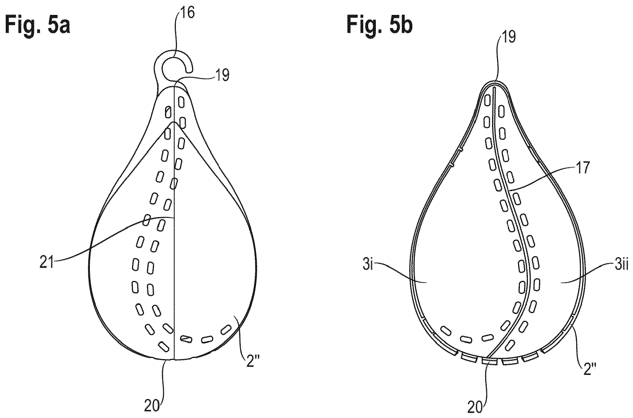

Fig. 5 shows the container as per Figure 4, likewise in front view (Fig 5a) in front view and the inner side view of container part 2' (Fig. 5b).

A fragrance release system indicated generally by 1 has a container indicated generally by 2 having chamber 3i and 3ii and a multitude of particles 4i and 4ii, stored in the respective chambers 3i and 3ii of the container 2, for deodorizing and/or fragrancing of closed spaces, in particular the interiors of machine dishwashers.

The particles 4i and 4ii have a carrier material, preferably made of a polymer, and also at least one fragrance, and preferably have a melting or softening point between 30° and 150°C, in particular 75°C and 80°C. Preferred materials for the particles 4i and 4ii are described in detail in the German patent publication DE10237066, which is explicitly incorporated by reference to avoid repetitions and whose disclosure content is incorporated into the disclosure content of this application.

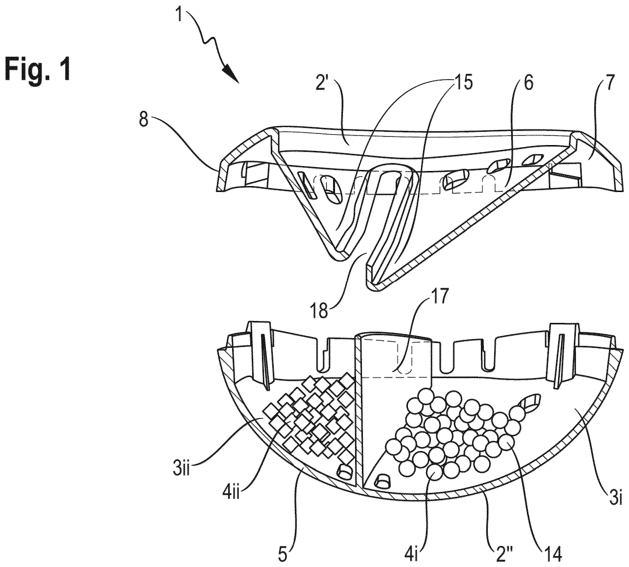

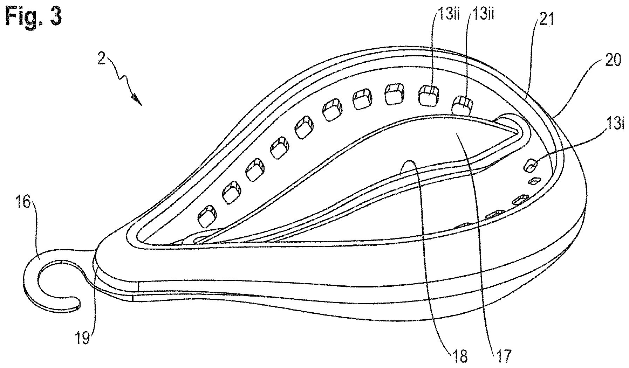

Important for the inventive fragrance release system 1 is the configuration of the container 2. As is best evident from Figures 1 to 3, the chambers 3i and 3ii of the container have together a crescent-like cross-sectional shape with a convex front wall 5 and a concave back wall 6, the end regions 7 of the crescent-like cross-sectional shape of the chambers 3i and 3ii being "rounded".

Preferably, as shown in the working examples, the container 2 has a two-part configuration, one part 2' having the back wall 6 and the other part 2" having the front wall 5.

The part 2', having the back wall 6, of the container 2 also has an edge region 8 which can cooperate with the edge region 9 of the other part 2", a snap-in connection preferably being provided for the connection. For instance, in the working example, a snap-in bead is provided on the edge region 9 and a snap-in lug on the interior of the edge region 8.

In the region of the convex front wall 5, the container 2 has a multitude of orifices 12i, 12ii, through which the emission of the fragrances of the particles 4i, 4ii from the chambers 3i, 3ii outward is possible. Orifices 13i, and 13ii, are on the back wall. In addition, a multitude of orifices 14i, 14ii are present in the region of the container which is the lowest region of the chambers 3i, 3ii when the container is use position, for drainage. The indices i and ii refer to the chamber first chamber (3i) and second chamber (3ii), respectively throughout the whole specification, e.g. the particles 4i are contained by the first chamber (3i); the orifices 13ii are on the back wall, on the part.

In the two-part configuration of the container 2 as shown, it is preferred that the concave back wall 6 curves inward. This provides a simplification of the filling operation. Further, for the embodiment it is preferred that the part 2" comprises a separator 17, which separator 17 separates the chamber 3i from the chamber 3ii. It is further preferred that the upper part 2' has a slot 18 in the inwardly curved wall, which slot 18 cooperates with the separator 17. Best filling is obtained when the concave portion of the wall 6 extends towards the front wall close to the separator 17.

In a preferred embodiment the fragrance release system 1 consists of perfumed polymer particles (e.g. ethylene/vinyl acetate copolymer) enclosed in a perforated container 2 (for example made of propylene). The polymer particles may be formed, for example, by Elvax^{®} 265 from Dupont having a melting point of 75°C, which can take up from about 25 to 30% perfume oil at room temperature. This granule is a common raw material for the plastics processing industry and is readily available. The fact that these particles can be laden with perfume oil at low temperature and combustion of the perfume oil can be avoided makes these particles particularly suitable for use in an inventive fragrance release system. The radius of the particles 4 is from about 1.5 to 3 mm and enables rapid economic loading with perfume oil.

In one preferred embodiment the two chambers have particles comprise different fragrance. Alternatively or in addition, the two chambers contain particles of a different color and/or shape, preferably a different color.

Both parts 2', 2" of the container 2 are preferably produced from polypropylene in an injection molding process; one of the two parts 2', 2" has on its exterior a hook 16 for hanging on a basket of a dishwasher or the like. The container parts 2', 2" may also be produced in another manner or consist of other plastics materials, as laid out in detail in the German patent application DE10237066, to which reference is explicitly made.

While the orifices 12i,12ii in the front wall 5 serve primarily for the emission of the perfume oil from the particles 4i, 4ii outward, for example into the interior of a machine dishwasher, the preferably orifices 13i,13ii, on the back wall 6 of the container are provided to enable water to run in and out in the course of a washing operation, in order to improve the fragrance release.

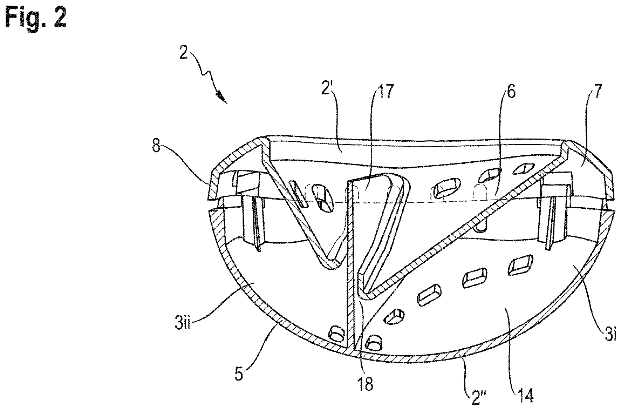

The chambers 3i, 3ii of the container 2 have, for example, a total volume of 40 ml, with a height and, following therefrom, a layer thickness of the particles 4i,4ii between about 10 and 15 mm. This chambers 3i,3ii are, as shown in Figures 1 and 2, fully filled with particles 4i,4ii. In the filling position (Figure 1), the part 2" having the front wall 5 forms the actual receptacle of the container 2, while the part 2' having the back wall 6 constitutes a kind of lid.

The required amount of particles 4i,4ii is initially introduced into the part 2", in such a way that the fill level remains somewhat below the upper edge of the edge region 9. Subsequently, the part 2' is laid on and lowered. About the first third of the concave back wall 6 with middle region 15 is immersed into the particles 4 and displaces some particles 4i, 4ii to the periphery of the container 2 and upward. Before the particles 4 reach the upper edge, the edge region 8 of the container part 2' reaches the edge region 9 of the part 2" and the particles 4i, 4ii cannot fall out of the container 2. The further lowering of the part 2' brings about the immersion of the further part of the middle region 15, which leads to particles being displaced from the peripheral region upward into the stillempty region of the chambers 3i, 3ii (small arrows in Figure 1), which results in this region of the chambers 3i, 3ii being filled fully. When this occurs, the two parts 2', 2" snap into one another owing to the snap-in connection, and the container 2 is closed permanently. In the case of a precise metered amount of particles 4i, 4ii, the container 2 is thus filled fully, as shown in Figure 2.

The particles 4i, 4ii are thus not arranged loosely in the container 2 and, after the first washing operation in a dishwasher, they form a conglomerate having a layer thickness of from 10 to 15 mm as a result of attainment of their softening temperature, which reduces the exposed surface area of the particles 4i, 4ii and enables a long functionality of the system. In contrast, if the particles 4i, 4ii were to be freely mobile within the container 2, the functioning time of the fragrance release system would be dependent only upon the amount of perfume oil which is present in each particle 4i, 4ii; the number of particles 4i, 4ii would only influence the fragrance intensity.

The inventive configuration of the fragrance release system thus reduces the exposed surface area of the particles 4i, 4ii, so that not all particles can release their fragrance at once; the fragrance intensity is thus determined by the amount of particles at the surface of the conglomerate in direct contact with the air, but not by all particles. The perfume oil from the inner particles migrates progressively to the surface of the conglomerate; the inner particles thus play a depot function for the fragrance release system and thereby enable a substantially longer functioning time of the system. It has been found that 50 washing operations at approx. 3 to 4 wash cycles per week using such a fragrance release system in a machine dishwasher are quite possible without impairment of function.

The above-described processes for designing the containers of inventive compositions will generally be directed just as much by visual considerations as the ultimate intended use of these compositions. The inventive compositions may, for example, in addition to the fragrance-containing particles, comprise further active substances. These active substances may be formulated within the container in a mixture or a blend with the fragrance-containing particles, or else separately from these particles. The active substances may also be incorporated into the container. These optional additional active substances may be used either in the form of an individual dose, for example for a single disinfection of a machine dishwasher, but also in the form of a multiple dosage.

In addition to the aforementioned active substances, it will be appreciated that the inventive compositions, especially compositions for use in machine dishwashers, textile washing machines or dryers, may comprise all active substances typically present in compositions for textile cleaning or dishwashing, or the care of textiles or dishes, particular preference being given to the group of the bleaches, bleach activators, polymers, builders, surfactants, enzymes, electrolytes, pH modifiers, fragrances, perfume carriers, dyes, hydrotropes, foam inhibitors, antiredeposition agents, optical brighteners, graying inhibitors, shrink preventaives, anticrease agents, dye transfer inhibitors, active antimicrobial ingredients, germicides, fungicides, antioxidants, corrosion inhibitors, antistats, repellent and impregnation agents, swelling and antislip agents, nonaqueous solvents, fabric softeners, protein hydrolyzates and UV absorbers. Such combination products are then suitable, in addition to repeated fragrancing, also for single or multiple care or cleaning of textiles or dishes.

As explained at the outset, spaces are deodorized and fragranced by means of the inventive compositions by introducing these compositions into a space and subsequently heating to temperatures between 20 and 110°C. The present application therefore further provides a method for deodorizing and fragrancing spaces, characterized in that an inventive fragrance release system is introduced into the space and is heated to temperatures between 30 and 80°C. If the inventive fragrance release system is used in an environment where the temperatures are below the melting or softening temperature for the material of the particles used, it may have to be activated by carrying out a heat treatment before use. In other embodiments, fragrance emission is already available at room temperature, this is advantageous when it is desired to let a consumer decide which fragrance to choose on point of sale. Possible use locations are therefore: restrooms, passenger cells, toilet bowls, trash cans, closets.

Suitable for fragrancing are generally all closed spaces, but in particular the interiors of buildings, vehicles or appliances, preferably such as textile washing machines, dryers or machine dishwashers.

The use of inventive fragrance release systems for deodorizing and fragrancing closed or open spaces is further provided by the present application.

### References used in the Figures

1 system
2 container parts
2', 2" container parts
3i, 3ii chambers
4, 4i, 4ii particles
5 convex front wall
6 concave back wall
7 two end rounded regions 7 of the cross-sectional shape of the chambers 3i and 3ii
8 edge region
9 edge region
12 multitude of orifices 12i, 12ii
13 multitude of orifices 13i, 13ii
14 multitude of orifices 14i, 14ii
15 middle region 15
16 hook
17 Separator
18 Slot
19 upper extremity
20 lower extremity
21 distance between 19 and 20

## Claims

1. A fragrance release system (1) comprising a container (2) and a multitude of particles (4i, 4ii) stored in chambers (3i, 3ii) of the container (2) for deodorizing and/or fragrancing open or closed spaces, especially textile washing machines, dryers or machine dishwashers, said particles (4i, 4ii) having a preferably polymeric carrier material and at least one fragrance, and said container (2) having a plurality of orifices (12i, 12ii, 13i, 13ii, 14i, 14ii) through which emission of the fragrances of the particles from the chamber outward is possible, **characterized in that** the chambers (3i, 3ii) comprise at least a first chamber (3i) and a second chamber (3ii), wherein the volume of the first chamber (3i) is larger than the volume of the second chamber (3ii), wherein the volume ratio of the first chamber (3i) to the second chamber (3ii) is between 1,3 and 2,3.

2. The fragrance release system as claimed in claim 1, wherein the first chamber (3i) and the second chamber (3ii) form, together, a cross section with a convex front wall (5) and a concave back wall (6).

3. The fragrance release system as claimed in any of the previous claims, wherein the container is elongated, comprising two extremities, namely an upper extremity 19 and a lower extremity 20, wherein the lower 20 extremity is opposite to the upper extremity 19.

4. The fragrance release system as claimed in claim 3, wherein the container has a mirror symmetry regarding its front view, along an axis connecting the two extremities (19,20).

5. The fragrance release system as claimed in any of the previous claims, wherein the container (2) is in two parts, one part (2') having the back wall (6) and the other part (2") having the front wall (5).

6. The fragrance release system as claimed in claim 5, wherein the part (2") has a separator (17) separating the first chamber (3i) from the second chamber (3ii).

7. The fragrance release system as claimed in claim 6, wherein the separator (17) is elongated and wherein the separator (17) is curved along this elongation.

8. The fragrance release system as claimed in claim 6, wherein the separator (17) is elongation between the extremities (19,20) according to claim 3.

9. The fragrance release system as in any of claims 6, 7, or 8, wherein the concave back wall (6) curves inward and has a slot (18) configured to cooperate with the separator (17) when two parts (2', 2") are connected and thus the container (2) is in closed configuration.

10. The fragrance release system as claimed in any of the previous claims, wherein, from the front view, the container (2) is asymmetric in regards to its rotation.

11. The fragrance release system as claimed in any of the previous claims, wherein the container is tapered in the direction of its upper extremity (19), such that the summed cross section of the chambers (3i, 3ii) increase from the upper extremity (19) towards the lower extremity (20), at least until half the distance between extremities (19, 20).

12. The fragrance release system as claimed in any of the previous claims, wherein the upper extremity (19) of the container (2) has on its exterior a fixture means, such as a hook (16) or other securing means such as adhesive surfaces or the like.

## Patentansprüche

1. Duftfreisetzungssystem (1), das einen Behälter (2) und eine Vielzahl von Partikeln (4i, 4ii) umfasst, die in Kammern (3i, 3ii) des Behälters (2) gelagert sind, zum Desodorieren und/oder Beduften offener oder geschlossener Räume, insbesondere Textilwaschmaschinen, Trockner oder Geschirrspülmaschinen, wobei die Partikel (4i, 4ii) ein bevorzugt polymeres Trägermaterial und mindestens einen Duft aufweisen und der Behälter (2) mehrere Öffnungen (12i, 12ii, 13i, 13ii, 14i, 14ii) aufweist, durch die eine Emission der Düfte der Partikel aus der Kammer nach außen möglich ist, **dadurch gekennzeichnet, dass**
die Kammern (3i, 3ii) mindestens eine erste Kammer (3i) und eine zweite Kammer (3ii) umfassen, wobei das Volumen der ersten Kammer (3i) größer ist als das Volumen der zweiten Kammer (3ii), wobei das Volumenverhältnis der ersten Kammer (3i) zu der zweiten Kammer (3ii) zwischen 1,3 und 2,3 liegt.

2. Duftfreisetzungssystem nach Anspruch 1, wobei die erste Kammer (3i) und die zweite Kammer (3ii) zusammen einen Querschnitt mit einer konvexen Vorderwand (5) und einer konkaven Rückwand (6) ausbilden.

3. Duftfreisetzungssystem nach einem der vorhergehenden Ansprüche, wobei der Behälter länglich ist und zwei Endpunkte umfasst, nämlich einen oberen Endpunkt 19 und einen unteren Endpunkt 20, wobei der untere 20 Endpunkt dem oberen Endpunkt 19 gegenüberliegt.

4. Duftfreisetzungssystem nach Anspruch 3, wobei der Behälter in Bezug auf seine Vorderansicht entlang einer Achse, die die zwei Endpunkte (19, 20) verbindet, eine Spiegelsymmetrie aufweist.

5. Duftfreisetzungssystem nach einem der vorhergehenden Ansprüche, wobei der Behälter (2) zweiteilig ist, wobei ein Teil (2') die Rückwand (6) aufweist und der andere Teil (2") die Vorderwand (5) aufweist.

6. Duftfreisetzungssystem nach Anspruch 5, wobei der Teil (2") einen Separator (17) aufweist, der die erste Kammer (3i) von der zweiten Kammer (3ii) separiert.

7. Duftfreisetzungssystem nach Anspruch 6, wobei der Separator (17) länglich ist und wobei der Separator (17) entlang dieser Verlängerung gekrümmt ist.

8. Duftfreisetzungssystem nach Anspruch 6, wobei der Separator (17) eine Verlängerung zwischen den Endpunkten (19, 20) nach Anspruch 3 ist.

9. Duftfreisetzungssystem nach einem der Ansprüche 6, 7 oder 8, wobei die konkave Rückwand (6) sich nach innen krümmt und einen Schlitz (18) aufweist, der konfiguriert ist, um mit dem Separator (17) zusammenzuwirken, wenn zwei Teile (2', 2") verbunden sind und somit der Behälter (2) in einer geschlossenen Konfiguration ist.

10. Duftfreisetzungssystem nach einem der vorhergehenden Ansprüche, wobei der Behälter (2), in der Vorderansicht, bezüglich seiner Drehung asymmetrisch ist.

11. Duftfreisetzungssystem nach einem der vorhergehenden Ansprüche, wobei der Behälter in der Richtung seines oberen Endpunktes (19) derart verjüngt ist, dass der summierte Querschnitt der Kammern (3i, 3ii) von dem oberen Endpunkt (19) zu dem unteren Endpunkt (20) hin zunimmt, mindestens bis zu der Hälfte des Abstands zwischen den Endpunkten (19, 20).

12. Duftfreisetzungssystem nach einem der vorhergehenden Ansprüche, wobei der obere Endpunkt (19) des Behälters (2) an seiner Außenseite ein Befestigungsmittel, wie etwa einen Haken (16) oder ein anderes Anbringungsmittel wie Klebstoffoberflächen oder dergleichen, aufweist.

## Revendications

1. Système de libération de parfum (1) comprenant un récipient (2) et une multitude de particules (4i, 4ii) stockées dans des chambres (3i, 3ii) du récipient (2) destiné à désodoriser et/ou parfumer des espaces ouverts ou fermés, en particulier des lavelinge pour textile, des sèche-linge ou des lave-vaisselle, lesdites particules (4i, 4ii) ayant un matériau de support polymère de préférence et au moins un parfum, et ledit récipient (2) comprenant une pluralité d'orifices (12i, 12ii, 13i, 13ii, 14i, 14ii) à travers lesquels l'émission de parfums des particules de la chambre vers l'extérieur est possible, **caractérisé en ce que**
les chambres (3i, 3ii) comprennent au moins une première chambre (3i) et une seconde chambre (3ii), le volume de la première chambre (3i) étant supérieur au volume de la seconde chambre (3ii), le rapport de volume de la première chambre (3i) à la seconde chambre (3ii) étant compris entre 1,3 et 2,3.

2. Système de libération de parfum selon la revendication 1, la première chambre (3i) et la seconde chambre (3ii) formant ensemble une section transversale comprenant une paroi avant convexe (5) et une paroi arrière concave (6).

3. Système de libération de parfum selon l'une quelconque des revendications précédentes, le récipient étant allongé, comprenant deux extrémités, à savoir une extrémité supérieure 19 et une extrémité inférieure 20, l'extrémité inférieure 20 étant opposée à l'extrémité supérieure 19.

4. Système de libération de parfum selon la revendication 3, le récipient ayant une symétrie miroir en ce qui concerne sa vue de face, le long d'un axe reliant les deux extrémités (19, 20).

5. Système de libération de parfum selon l'une quelconque des revendications précédentes, le récipient (2) étant en deux parties, une partie (2') comprenant la paroi arrière (6) et l'autre partie (2") comprenant la paroi avant (5).

6. Système de libération de parfum selon la revendication 5, la partie (2") ayant un séparateur (17) séparant la première chambre (3i) de la seconde chambre (3ii).

7. Système de libération de parfum selon la revendication 6, le séparateur (17) étant allongé et le séparateur (17) étant incurvé le long de cet allongement.

8. Système de libération de parfum selon la revendication 6, le séparateur (17) étant un allongement entre les extrémités (19, 20) selon la revendication 3.

9. Système de libération de parfum selon l'une quelconque des revendications 6, 7 ou 8, la paroi arrière concave (6) s'incurvant vers l'intérieur et ayant une fente (18) conçue pour coopérer avec le séparateur (17) lorsque deux parties (2', 2") sont reliées et ainsi le récipient (2) étant en configuration fermée.

10. Système de libération de parfum selon l'une quelconque des revendications précédentes, depuis la vue de face, le récipient (2) étant asymétrique en ce qui concerne sa rotation.

11. Système de libération de parfum selon l'une quelconque des revendications précédentes, le récipient étant effilé dans la direction de son extrémité supérieure (19), de telle sorte que la section transversale additionnée des chambres (3i, 3ii) augmente à partir de l'extrémité supérieure (19) vers l'extrémité inférieure (20), au moins jusqu'à la moitié de la distance entre les extrémités (19, 20).

12. Système de libération de parfum selon l'une quelconque des revendications précédentes, l'extrémité supérieure (19) du récipient (2) comprenant sur son extérieur un moyen de fixation, tel qu'un crochet (16) ou d'autres moyens de fixation tels que des surfaces adhésives ou similaire.
